# FASCICULE DE BREVET EUROPEEN

(11) **EP 2 483 420 B1**
(45) Date de publication et mention de la délivrance du brevet: **25.06.2014**
(21) Numéro de dépôt: 10759682.7
(22) Date de dépôt: 30.09.2010
(51) Int. Cl.: C12Q 1/68

(54) **METHODE DE DETECTION DE MICROORGANISMES**
VERFAHREN FÜR DEN NACHWEIS VON MIKROORGANISMEN
METHOD FOR DETECTING MICROORGANISMS

(30) Priorité: 30.09.2009 FR 0904677
(43) Date de publication de la demande: 08.08.2012
(73) Titulaire: Assistance Publique Hôpitaux De Paris, 75004 Paris (FR); Institut Pasteur, 75015 Paris (FR); Université Paris Diderot - Paris 7, 75205 Paris Cedex 13 (FR)
(72) Inventeur: RUPPE, Etienne, 75015 Paris (FR); ANDREMONT, Antoine, 92240 Malakoff (FR); RUIMY, Raymond, 95370 Montigny les Cormeilles (FR); COURVALIN, Patrice, 75015 Paris (FR); BREMONT, Sylvie, 93130 Noisy Le Sec (FR)
(74) Mandataire: Flesselles, Bruno F.G.
(86) Numéro de dépôt international: PCT/EP2010/064589
(87) Numéro de publication internationale: WO 2011/039321

(56) Documents cités:
- WO-A2-01/23604
- US-A1- 2004 002 080
- PEREZ-PEREZ F JAVIER ET AL: "Detection of plasmid-mediated AmpC beta-lactamase genes in clinical isolates by using multiplex PCR" JOURNAL OF CLINICAL MICROBIOLOGY, AMERICAN SOCIETY FOR MICROBIOLOGY, WASHINGTON, DC, US, vol. 40, no. 6, 1 juin 2002 (2002-06-01), pages 2153-2162, XP002420840 ISSN: 0095-1137
- DATABASE EMBL [Online] 20 février 2003 (2003-02-20), "Sequence 2982 from Patent WO02053774." XP002568755 extrait de EBI accession no. EMBL:AX625941 Database accession no. AX625941
- DATABASE Geneseq [Online] 22 février 2002 (2002-02-22), "Oligonucleotide primer SEQ ID NO 360790 for detecting SNP TSC0052292." XP002568756 extrait de EBI accession no. GSN:ABI60817 Database accession no. ABI60817
- KOH ET AL: "Emerging problems with plasmid-mediated DHA and CMY AmpC beta-lactamases in Enterobacteriaceae in Singapore" INTERNATIONAL JOURNAL OF ANTIMICROBIAL AGENTS, ELSEVIER SCIENCE, AMSTERDAM, NL, vol. 30, no. 3, 1 septembre 2007 (2007-09-01), pages 278-280, XP022181953 ISSN: 0924-8579 & DATABASE EMBL [Online] 21 février 2007 (2007-02-21), "Escherichia coli plasmid AmpC beta-lactamase CMY-2 (cmy-2) gene, complete cds." extrait de EBI accession no. EMBL:EF406116 Database accession no. EF406116
- BARLOW MIRIAM ET AL: "Origin and evolution of the AmpC beta-lactamases of Citrobacter freundii" ANTIMICROBIAL AGENTS AND CHEMOTHERAPY, vol. 46, no. 5, mai 2002 (2002-05), pages 1190-1198, XP002568757 ISSN: 0066-4804
- PHILIPPON ALAIN ET AL: "Plasmid-determined AmpC-type beta-lactamases" ANTIMICROBIAL AGENTS AND CHEMOTHERAPY, vol. 46, no. 1, janvier 2002 (2002-01), pages 1-11, XP002568758 ISSN: 0066-4804
- WESTIN L ET AL: "ANTIMICROBIAL RESISTANCE AND BACTERIAL IDENTIFICATON UTILIZING A MICROELECTRONIC CHIP ARRAY" JOURNAL OF CLINICAL MICROBIOLOGY, AMERICAN SOCIETY FOR MICROBIOLOGY, WASHINGTON, DC, US, vol. 39, no. 3, 1 mars 2001 (2001-03-01), pages 1097-1104, XP001006185 ISSN: 0095-1137

## Description

L'invention se rapporte au domaine du diagnostic et à la détection de bactéries résistantes aux antibiotiques dans un échantillon.

L'activité des antibiotiques tend à se réduire au fil du temps du fait de l'apparition et de la diffusion de mécanisme de résistance bactériens. Aujourd'hui la mise sur le marché de nouvelles molécules antibactériennes efficaces est extrêmement ralentie, alors que l'usage extensif en médecine humaine et vétérinaire des antibiotiques a conduit à la diffusion massive et globalisée des déterminants de la résistance bactérienne.

Les β-lactamines constituent la famille d'antibiotiques la plus consommée dans le monde du fait de leur spectre d'activité antibactérienne large, leur efficacité et leur quasi-absence d'effets indésirables. Par conséquent, les mécanismes de résistance à ces antibiotiques sont largement répandus dans le monde bactérien, notamment parmi les bactéries pathogènes que sont les entérobactéries.

Le mécanisme de résistance aux β-lactamines le plus répandu parmi elles est la production d'enzymes hydrolysantes appelées β-lactamases. Ces enzymes constituent une famille très hétérogène en terme de spectre d'hydrolyse et de séquence nucléotidique. Les gènes codant pour ces enzymes sont situés dans le chromosome des bactéries ou sur des éléments mobiles comme les plasmides. Ce support transmissible de bactérie à bactérie assure au gène de résistance une diffusion importante. Si l'origine de la plupart des β-lactamases demeure inconnue à ce jour, on a pu observer récemment la mobilisation de gènes d'origine chromosomiques sur des éléments mobiles, dont les céphalosporinases plasmidiques.

Les céphalosporinases ont un spectre d'hydrolyse large, et le traitement des infections causées par des bactéries productrices de telles enzymes est restreint à quelques molécules (carbapénèmes), dont l'activité peut être compromise s'il existe d'autres mécanismes de résistance.

La diffusion des gènes de céphalosporinases chez les bactéries pathogènes comme *Escherichia coli* est un problème majeur de Santé Publique, au même titre que la diffusion des β-lactamases à spectre élargi (BLSE). Le seul moyen efficace de combattre cette diffusion au sein de structures de santé est de détecter les patients porteurs de bactéries productrices de telles enzymes et de prendre les mesures d'hygiène et de confinement nécessaires pour limiter les transmissions de patient à patient. La détection de bactéries multirésistantes dans la flore nasale (*Staphylococcus aureus* résistant à la méticilline, ou SARM) ou digestive (entérobactéries productrices de BLSE, entérocoque résistant à la vancomycine, ou ERV) est aujourd'hui une pratique de routine dans de nombreuses structures de soins. Cependant la plupart des bactéries productrices de céphalosporinases plasmidiques ne sont pas reconnues par ces méthodes, laissant place à la diffusion invisible de ces gènes.

De nombreux auteurs ont proposés diverses méthodes phénotypiques pour mettre en évidence la production de céphalosporinases au sein de souches bactériennes (1, 3-11). Toutefois, la position chromosomique ou plasmidique du gène de résistance (capitale en terme d'impact de diffusion et donc en terme de mesures d'hygiène à mettre en oeuvre) ne peut être déterminée que par des méthodes moléculaires pour les bactéries susceptibles de produire leur propre céphalosporinase chromosomique. En effet les séquences nucléotidiques sont virtuellement identiques entre les gènes en position plasmidiques et les gènes chromosomiques. Dès lors si la bactérie « parentale » est fréquemment présente dans la flore des patients, le risque est d'aboutir à un grand nombre de faux positifs quant à la détection des céphalosporinases plasmidiques.

Perez-Perez et Hanson (J Clin Microbiol. 2002 Jun;40(6):2153-62**) décrivent un procédé de détermination de l'origine plasmidique d'un gène CMY-2 porté par une bactérie comprenant une étape d'amplification par PCR. Ce procédé ne permet pas de distinguer entre l'origine chromosomique ou plasmidique du gène en présence d'une bactérie "parentale" ayant le même gène chromosomique (*Citrobacter freundii*).**

La présente invention fournit une méthode permettant de détecter la présence, dans un échantillon, d'une bactérie portant un gène plasmidique de céphalosporinase de type CMY-2, en en présence ou non de la bactérie « parentale » *Citrobacter freundii.*

Cette détection est maintenant possible par la mise en évidence par les inventeurs que le gène CMY-2 d'origine plasmidique présente une cytosine (resp une guanine) en position 373 (resp 437), le gène d'origine chromosomique présentant une adénosine (resp une cytosine).

La méthode de l'invention permet ainsi de différentier et caractériser la localisation (plasmidique ou chromosomique) du gène CMY-2 : l'analyse spécifique des deux nucléotides mentionnés ci-dessus permet de discriminer les souches qui portent le gène ciblé en position plasmidique de celles qui le portent en position chromosomique.

L'invention se rapporte ainsi à une méthode de détection de la présence d'au moins une bactérie résistante aux β-lactamines dans un échantillon, et porteuse d'un gène CMY-2 plasmidique, caractérisée en ce qu'elle comprend l'étape de détection de la présence d'une molécule d'ADN dans ledit échantillon, dont la séquence présente au moins 90 % d'identité, de préférence au moins 95 %, de façon plus préférée au moins 98 %, de façon la plus préférée au moins 99 % d'identitié avec SEQ ID N° 1, et dont les nucléotides 373 et 437 sont respectivement une cytosine et une guanine.

SEQ ID N° 1 représente ainsi un allèle plasmidique du gène CMY-2. Sa séquence peut varier du fait de la diversité génétique. SEQ ID N° 1 correspond à l'accession GenBank AM779745, correspondant au gène blaCMY-2 pour la bêta-lactamase CMY-2, isolé du plasmide pta d'*Escherichia coli.*

D'autres gènes plasmidiques CMY-2 sont également disponibles dans les bases de données, sous divers numéros d'accession.

La présence de ces deux nucléotides (C373 et G437) dans le gène CMY-2 est indicative de la nature plasmidique du gène. En effet, les gènes d'origine chromosomique présentent respectivement une adénosine et une cytosine.

La conséquence de ces mutations est la présence d'une arginine en position 125 ou 146 dans les protéines exprimées par le gène plasmidique, les gènes d'origine chromosomique exprimant une protéine comprenant une sérine en position 125 et une thréonine en position 146.

Il convient de noter que la méthode selon l'invention permet théoriquement de détecter la présence d'une seule bactérie contenant un CMY-2 plasmidique dans l'échantillon (avec des conditions d'amplification satisfaisantes).

Toutefois, la méthode selon l'invention ne permet pas de déterminer si les bactéries porteuses du gène CMY-2 plasmidique sont toutes du même genre ou s'il existe différents genres de bactéries contenant le plasmide dans l'échantillon. Des tests complémentaires sont nécessaires pour préciser ce point et caractériser la nature de la bactérie porteuse de la résistance.

L'échantillon peut être de tout type. In peut s'agit d'un échantillon biologique qui a préalablement été prélevé sur un patient. Un tel échantillon peut être, sans que cette liste soit exhaustive, un échantillon de salive un échantillon urinaire, fécal, sanguin, ou issu d'une biopsie, notamment d'une biopsie intestinale.

La méthode peut également être pratiquée sur un prélèvement réalisé sur un échantillon alimentaire, sur une surface ou sur un ustensile, notamment utilisable en milieu hospitalier (vérification de l'asepsie de la surface ou de l'ustensile).

La méthode selon l'invention permet ainsi de déterminer la présence de bactéries contenant un gène CMY-2 d'origine plasmidique, y compris lorsque l'échantillon contient des bactéries du genre *Citrobacter freundii.*

En effet, il est en effet probable que le gène CMY-2 plasmidique soit dérivé du gène chromosomal AmpC de *C. freundii* (2). Ce gène AmpC présente une séquence proche de SEQ ID N° 1 (identité supérieure à 80 % avec SEQ ID N° 1, et pouvant être supérieure à 90 % pour certains allèles), mais dont les nucléotides 373 et 437 sont respectivement une adénosine et une cytosine. Il convient de noter qu'il existe la variabilité existant entre les différents gènes chromosomiques est plus importante que celle existant entre les différents gènes plasmidiques.

Le pourcentage d'identité entre deux séquences de nucléotides est déterminé en comparant les deux séquences alignées de manière optimale, sur une fenêtre de comparaison.

Dans le cadre de la présente invention, on aligne ainsi les deux séquences sur l'ensemble des 1143 nucléotides correspondant à la séquence codant pour la protéine de résistance aux β-lactamines (1146 en comptant le codon stop final). Le pourcentage d'identité est ainsi calculé par la formule suivante : pourcentage d'identité = ((nombre de différences entre les deux séquences) / 1143) x 100.

Des algorithmes connus peuvent permettre de détecter les différences entre deux séquences de façon automatisée, et de mesurer ainsi le pourcentage d'identité.

Dans un mode de réalisation préféré, ladite étape de détection des polymorphismes comprend une étape d'amplification de l'ADN présent dans ledit échantillon. Ceci permet en effet de détecter la présence d'un nombre très faible de bactéries.

Cette amplification est réalisée par réaction de polymérisation en chaîne PCR en utilisant toute amorce pouvant être dessinée par l'homme du métier.

On utilise de préférence les amorces Citro strt F (5'-ATGATGAAAAAATCGATATGCTGCG-3', positions 1-25, SEQ ID N° 2) et Citro_strt_R (5'-CAAACAGACCAATGCTGGAGTTAGC-3', positions 511-535, SEQ ID N° 3) amplifiant un fragment de 535 pb.

On peut également utiliser les amorces Citro_F (5'-TGCTGCTGACAGCCTCTTTCTCC-3', positions 29-51, SEQ ID N° 4) et Citro_R (5'- GGCGGGTTTACCTCAACGGC-3', positions 952-971, SEQ ID N° 5), amplifiant un fragment de 942 pb.

On peut également utiliser le couple d'amorces SEQ ID N° 2 - SEQ ID N° 5.

L'homme du métier est à même de dessiner d'autres types d'amorces pour amplifier le gène CMY-2. Toutefois, les inventeurs ont montré que les amorces ci-dessus présentent une bonne efficacité.

On peut effectuer une amplification avec un seul couple d'amorces, ou effectuer deux séries de PCR, la seconde amplification étant réalisée avec des amorces situées à l'intérieur du premier fragment amplifié.

On utilise de préférence une ADN polymérase à haute ou très haute fidélité. En effet, comme on recherche la présence spécifique de nucléotides définis, il est important que la polymérase n'induise pas de mutations dans le fragment amplifié. De telles enzymes sont disponibles commercialement. On peut ainsi citer les enzymes Phusion™ DNA Polymerases (Finnzymes Oy, Espoo, Finlande) présentant un taux d'erreur de l'ordre de 4,4 x 10⁻⁷.

Lors de la mise en oeuvre de la réaction d'amplification, on peut choisir de n'amplifier que le gène CMY-2, en utilisant un coupe d'amorces approprié, ou d'amplifier, dans la même réaction, différents gènes potentiellement présents dans l'échantillon (notamment d'autres gènes de résistances à divers antibiotiques), en utilisant une pluralité d'amorces différentes. On introduit ainsi dans le tube de réaction plusieurs couples d'amorces, chacun étant spécifique d'un gène que l'on souhaite amplifier.

La détection des nucléotides polymorphes, indicateurs de la présence du gène CMY-2 plasmidique s'effectue de toute manière connue dans l'art. L'homme du métier connaît en effet de multiples protocoles appropriés qui peuvent être identifiés sur des moteurs de recherche sur Internet. On peut notamment citer www.ipbs.fr/formation/biotech/mutations.pdf.

On peut ainsi séquencer le fragment d'ADN, en particulier par la méthode de séquençage de Sanger en utilisant uniquement trois désoxynucléotides et un didésoxynucléotide et une amorce (notamment une amorce telle que mentionnée ci-dessus). Les produits de séquence sont ensuite séparés sur gel de polyacrylamide et la présence de la mutation est confirmée par observation de la taille des produits.

On peut aussi faire une réaction d'extension d'amorce. Dans ce cas, l'amorce utilisée est définie de telle sorte que le premier nucléotide à incorporer après cette amorce corresponde au nucléotide polymorphe.

Toutefois, d'une façon préférée, ladite étape de détection comprend une étape d'hybridation de l'ADN (préférentiellement amplifié) avec une sonde spécifique de l'une ou l'autre mutation, ou des deux mutations. L'hybridation de deux chaînes d'ADN est due à l'appariement de bases complémentaires A-T et G-C et est diminuée lors de mésappariement. La Tm d'un oligonucléotide de 10 à 20 bases sera donc abaissé s'il y a un mésappariement. Pour calculer le Tm d'un oligonucléotide inférieur à 30 nucléotides, on utilise la relation suivante : Tm = 2 (A + T) + 4 (G + C), dans laquelle A, T, G et C sont respectivement le nombre de chacune de ces bases dans l'oligonucléotide.

On peut ainsi utiliser la méthode « dot blot », en fixant un oligonucléotide sonde porteur de la mutation sur une membrane de nitrocellulose, et en appliquant l'ADN présent dans l'échantillon (ou l'ADN amplifié) dans des conditions permettant l'hybridation spécifique du polymorphisme (la TM est calculée selon la composition de l'oligonucléotide sonde choisi.

Dans ce cas, la sonde est fixée à un support solide.

Dans un mode de réalisation particulier, l'hybridation est réalisée sur une puce à ADN. Un premier spot contient un oligonucléotide présentant le polymorphisme du gène AmpC chromosomal de *Citrobacter freundii,* et un deuxième spot contient un oligonucléotide présentant le polymorphisme du gène CMY-2 plasmidique. On amplifie et et marque l'ADN avec un fluorophore. Il est ensuite hybridé sur les oligonucléotides fixés à la puce. On observe une fluorescence s'il y a hybridation.

On peut aussi détecter la présence des deux nucléotides mentionnés plus haut par d'autres méthodes. Après amplification, on peut mélanger le produit d'amplification avec de l'ADN ne comprenant pas les mutations et hybrider ces deux oligonucléotides. Les mésappariements sont alors détectés par coupure enzymatique (notamment en utilisation de *Cel*I) ou chimique. Cette méthode de détection est bien connue et des enzymes appropriées sont notamment décrites dans EP 964929.

L'invention se rapporte également à un kit pour la détection de la présence d'une au moins une bactérie résistante aux β-lactamines dans un échantillon, ladite bactérie possédant un gène CMY-2 plasmidique.

Le kit selon l'invention **comprend** des amorces permettant d'amplifier le gène CMY-2. Il **comprend** également **une sonde** permettant d'identifier la présence d'une cytosine en position 373 et/ou d'une guanine en position 437, les positions étant déterminée par rapport à SEQ ID N° 1. Les sondes peuvent être présentées sous forme déshydratées. Alternativement, le kit peut contenir une puce à ADN comprenant lesdites sondes. Le kit peut également contenir des enzymes permettant l'amplification de l'ADN cible, des éléments permettant le marquage de l'ADN. De préférence, le kit selon l'invention contient également des instructions pour la mise en oeuvre de la méthode telle que décrite ci-dessus.

### Exemples

### Exemple 1

On a recherché la nature des nucléotides 373 et 437 du gène chromosomique *ampC* de 83 souches de *C. freundii* d'origines variées : 62 souches cliniques de l'hôpital Bichat-Claude Bernard (Assistance Publique des Hôpitaux de Paris) isolées entre octobre 2006 et janvier 2009, 18 souches de l'Unité des Agents Antimicrobiens de l'Institut Pasteur de Paris, 4 souches isolées de la flore digestive d'Indiens WaYampis vivant en Guyane Française (projet « ERAES », AFFSET).

Ces souches ont été identifiées comme *C. freundii* par les méthodes conventionnelles.

On a aussi étudié les nucléotides des gènes *ampC* présents dans les bases de données (numéros d'accession GenBank) : CFRBL, CFRBLAC, X03866, X51632, Y15129, X76636, AF349569, AF349570, AF746169, AF125469, AF426097.

Le gène ampC de *C. freundii* compte 1143 paires de bases (pb) (sans prendre en compte le codon STOP).

L'analyse de ces gènes montre que le nucléotide 373 de chacun de ces gènes est une adénosine. Le nucléotide 437 de chacun de ces gènes est une cytosine.

De façon inverse, tous les gènes présentant une cytosine à la position 373 et d'une guanine en position 437 sont d'origine plasmidique : voir notamment les gènes suivants (numéros d'accession GenBank) AM779745, AM779746, AM779747, AM779748, AY899923, AY899924, AY899925, AY899926, AY899927, AY899928, AY899929, DQ173299, DQ355981, DQ478718, EF406116, EU113220, EU113221, EU113222.

Ces résultats montrent que la présence des nucléotides C373 et G437 dans un gène CMY-2 indique qu'il est d'origine plasmidique.

## Revendications

1. Méthode de détermination de l'origine d'un gène CMY-2 porté par une bactérie, dans un échantillon, **caractérisée en ce qu'**elle comprend l'étape de recherche de la nature des nucléotides 373 et 437 dans ledit gène CMY-2, un gène CMY-2 d'origine plasmidique présentant une cytosine (resp une guanine) en position 373 (resp 437), un gène CMY-2 d'origine chromosomique présentant une adénosine (resp une cytosine), les positions étant déterminées par rapport à SEQ ID NO: 1.

2. Méthode selon la revendication 1, **caractérisée en ce que** ledit échantillon contient des bactéries du genre *Citrobacter freundii.*

3. Méthode selon l'une des revendications 1 ou 2, **caractérisée en ce qu'**elle comprend une étape d'amplification de l'ADN présent dans ledit échantillon.

4. Méthode selon l'une des revendications 1 à 3, **caractérisée en ce qu'**elle comprend une étape d'hybridation de l'ADN avec une sonde spécifique de l'une ou l'autre mutation.

5. Méthode selon la revendication 4, **caractérisée en ce que** ladite sonde est fixée à un support solide.

6. Kit pour la détection de la présence d'une bactérie résistante aux β-lactamines, et porteuse d'un gène CMY-2 plasmidique, dans un échantillon, comprenant des instructions pour la mise en oeuvre d'une méthode selon l'une des revendications 1 à 5, ainsi qu'une sonde permettant d'identifier la présence d'une cytosine en position 373 et/ou d'une guanine en position 437, les positions étant déterminées par rapport à SEQ ID N° 1, ledit kit contenant également des amorces permettant d'amplifier le gène CMY-2.

## Patentansprüche

1. Verfahren zum Bestimmen des Ursprungs eines von einem Bakterium geborgenen CMY-2-Gens in einer Probe, **dadurch gekennzeichnet, dass** es den Schritt umfasst, dass man die Art der Nukleotide 373 und 437 in dem CMY-2-Gen, untersucht, wobei ein CMY-2-Gen plasmidischen Ursprungs ein Cytosin (bzw. ein Guanin) in Position 373 (bzw. 437) aufweist, während ein CMY-2-Gen chromosomalen Ursprungs ein Adenosin (bzw. ein Cytosin) aufweist, wobei die Positionen in Bezug auf SEQ ID NO: 1 angegeben sind.

2. Verfahren nach Anspruch 1, **dadurch gekennzeichnet, dass** die Probe Bakterien der Gattung *Citrobacter freundii* enthält.

3. Verfahren nach einem der Ansprüche 1 oder 2, **dadurch gekennzeichnet, dass** es einen Amplifikationsschritt der in der Probe vorhandenen DNA umfasst.

4. Verfahren nach einem der Ansprüche 1 bis 3, **dadurch gekennzeichnet, dass** es einen Hybridisierungsschritt der DNA mit einer für die eine oder andere Mutation spezifischen Sonde umfasst.

5. Verfahren nach Anspruch 4, **dadurch gekennzeichnet, dass** die Sonde auf einem festen Träger fixiert ist.

6. Kit zum Nachweisen eines β-lactaminresistenten Bakteriums, das ein plasmidisches CMY-2-Gen trägt, in einer Probe, das Anweisungen für das Durchführen eines Verfahrens nach einem der Ansprüche 1 bis 5 sowie eine Sonde, die das Vorhandensein eines Cytosins in Position 373 und/oder eines Guanins in Position 437, wobei die Positionen in Bezug auf SEQ ID NO: 1 festgelegt sind, gestattet, umfasst, wobei das Kit zusätzlich Matrizen, mit denen das CMY-2-Gen amplifiziert werden kann, umfasst.

## Claims

1. Method for determining the origin of a CMY-2 gene carried by a bacterium, in a sample, **characterized in that** it comprises the step of investigating the nature of nucleotides 373 and 437 in said CMY-2 gene, a CMY-2 gene of plasmid origin having a cytosine (respectively a guanine) in position 373 (respectively 437), a CMY-2 gene of chromosomal origin having an adenosine (respectively a cytosine), the positions being determined relative to SEQ ID No. 1.

2. Method according to Claim 1, **characterized in that** said sample contains bacteria of the genus *Citrobacter freundii.*

3. Method according to either of Claims 1 and 2, **characterized in that** it comprises a step of amplification of the DNA present in said sample.

4. Method according to one of Claims 1 to 3, **characterized in that** it comprises a step of hybridization of the DNA with a probe specific for either mutation.

5. Method according to Claim 4, **characterized in that** said probe is attached to a solid support.

6. Kit for detecting the presence, in a sample, of a bacterium which is resistant to β-lactam antibiotics and which carries a plasmid CMY-2 gene, comprising instructions for carrying out a method according to one of Claims 1 to 5, and also a probe for identifying the presence of a cytosine in position 373 and/or a guanine in position 437, the positions being determined relative to SEQ ID No. 1, said kit also containing primers for amplifying the CMY-2 gene.


### SEQUENCE LISTING

<110> Assistance Publique - Hôpitaux de Paris Institut Pasteur
<120> Méthode de détection de microorganismes
<130> BRV 17 - WO
<150> FR 09/04677
   <151> 2009-09-30
<160> 5
<170> PatentIn version 3.3
<210> 1
   <211> 1146
   <212> DNA
   <213> Escherichia coli
<400> 1
   atgatgaaaa aatcgttatg ctgcgctctg ctgctgacag cctctttctc cacatttgct 60
   gccgcaaaaa cagaacaaca gattgccgat atcgttaatc gcaccatcac cccgttgatg 120
   caggagcagg ctattccggg tatggccgtt gccgttatct accagggaaa accctattat 180
   ttcacctggg gtaaagccga tatcgccaat aaccacccag tcacgcagca aacgctgttt 240
   gagctaggat cggttagtaa gacgtttaac ggcgtgttgg gcggcgatgc tatcgcccgc 300
   ggcgaaatta agctcagcga tccggtcacg aaatactggc cagaactgac aggcaaacag 360
   tggcagggta tccgcctgct gcacttagcc acctatacgg caggcggcct accgctgcag 420
   atccccgatg acgttaggga taaagccgca ttactgcatt tttatcaaaa ctggcagccg 480
   caatggactc cgggcgctaa gcgactttac gctaactcca gcattggtct gtttggcgcg 540
   ctggcggtga aaccctcagg aatgagttac gaagaggcaa tgaccagacg cgtcctgcaa 600
   ccattaaaac tggcgcatac ctggattacg gttccgcaga acgaacaaaa agattatgcc 660
   tggggctatc gcgaagggaa gcccgtacac gtttctccgg gacaacttga cgccgaagcc 720
   tatggcgtga aatccagcgt tattgatatg gcccgctggg ttcaggccaa catggatgcc 780
   agccacgttc aggagaaaac gctccagcag ggcattgcgc ttgcgcagtc tcgctactgg 840
   cgtattggcg atatgtacca gggattaggc tgggagatgc tgaactggcc gctgaaagct 900
   gattcgatca tcaacggcag cgacagcaaa gtggcattgg cagcgcttcc cgccgttgag 960
   gtaaacccgc ccgcccccgc agtgaaagcc tcatgggtgc ataaaacggg ctccactggt 1020
   ggatttggca gctacgtagc cttcgttcca gaaaaaaacc ttggcatcgt gatgctggca 1080
   aacaaaagct atcctaaccc tgtccgtgtc gaggcggcct ggcgcattct tgaaaagctg 1140
   caataa 1146
<210> 2
   <211> 25
   <212> DNA
   <213> Artificial
<220>
   <223> amorce Citro strt F
<400> 2
   atgatgaaaa aatcgatatg ctgcg 25
<210> 3
   <211> 25
   <212> DNA
   <213> Artificial
<220>
   <223> amorce Citro_strt_R
<400> 3
   caaacagacc aatgctggag ttagc 25
<210> 4
   <211> 23
   <212> DNA
   <213> Artificial
<220>
   <223> amorce Citro_F
<400> 4
   tgctgctgac agcctctttc tcc 23
<210> 5
   <211> 20
   <212> DNA
   <213> Artificial
<220>
   <223> amorce Citro_R
<400> 5
   ggcgggttta cctcaacggc 20